# EUROPÄISCHE PATENTSCHRIFT

(11) **EP 1 354 867 B1**
(45) Veröffentlichungstag und Bekanntmachung des Hinweises auf die Patenterteilung: **01.04.2015**
(21) Anmeldenummer: 03008409.9
(22) Anmeldetag: 11.04.2003
(51) Int. Cl.: C07C 69/78, C08K 5/101

(54) **Benzoesäureisononylester und deren Verwendung**
Benzoic acid isononyl esters and their use
Esters isononyliques d'acide benzoique et leur utilisation

(30) Priorität: 18.04.2002 DE 10217186
(43) Veröffentlichungstag der Anmeldung: 22.10.2003
(73) Patentinhaber: Evonik Degussa GmbH, 45128 Essen (DE)
(72) Erfinder: Grass, Michael, Dr., 45721 Haltern am See (DE); Koch, Jürgen, 45721 Haltern am See (DE)
(74) Vertreter: Hirsch, Hans-Ludwig

(56) Entgegenhaltungen:
- CH-A- 274 531
- DE-A- 1 962 500
- DATABASE CAPLUS [Online] CHEMICAL ABSTRACTS SERVICE, COLUMBUS, OHIO, US; MURAVLYANSKAYA, T. B. ET AL: "Comparative characteristics of plasticizers" retrieved from STN Database accession no. 74:23306 XP002258318 & PLASTICHESKIE MASSY (1970), (10), 18-20 ,
- DATABASE REGISTRY [Online] CHEMICAL ABSTRACTS SERVICE, COLUMBUS, OHOI, US; Database accession no. 91994-92-2 XP002258319
- DATABASE REGISTRY [Online] CHEMICAL ABSTRACTS SERVICE, COLUMBUS, OHIO, US; Database accession no. 68526-83-0 XP002258320

## Beschreibung

Die Erfindung betrifft isomere Nonylbenzoate, Verfahren zu deren Herstellung, deren Gemische mit Phthalsäurealkylestern, Adipinsäurealkylestern oder Cyclohexandicarbonsäurealkylestern sowie die Verwendung dieser Gemische.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden. Mit zunehmender Kettenlänge der Ester steigen die Löse- bzw. Geliertemperaturen und somit die Verarbeitungstemperaturen des Weich-PVC an. Die Verarbeitungstemperaturen können durch Zusatz von sogenannten Schnellgelierern wie beispielsweise die kurzkettigen Phthalate Dibutylphthalat (DBP), Diisobutylphthalat (DIBP), Benzylbutylphthalat (BBP) oder Diisoheptylphthalat (DIHP) wieder reduziert werden. Neben den kurzkettigen Phthalaten können auch Dibenzoesäureester wie Dipropylenglycoldibenzoate o.ä. zum gleichen Zwecke eingesetzt werden.
Diese Schnellgelierer zeigen oftmals die Eigenschaft, in PVC-Plastisolen auf Grund ihrer hohen Solvatationskraft zu einem starken Viskositätsanstieg mit der Zeit zu führen. Dies muss in vielen Fällen wieder durch Zugabe von (oft teuren) viskositätsreduzierenden Agenzien kompensiert werden.

Bei der Herstellung von PVC-Plastisolen ist in der Regel eine niedrige Viskosität und eine möglichst niedrige Geliertemperatur gefordert. Darüber hinaus wird eine hohe Lagerstabilität (geringer Viskositätsanstieg mit der Zeit) des Plastisols gewünscht.
Eine hohe Viskosität wäre bei der maschinellen Verarbeitung des Plastisols nachteilig; eine zu hohe Geliertemperatur würde zu Verfärbungen durch die thermische Belastung führen.

Weichmacher, die sowohl die Geliertemperatur in einer Formulierung signifikant absenken als auch die Viskosität des Plastisols auch nach mehrtägiger Lagerdauer auf einem niedrigen Niveau halten, sind bisher kaum bekannt. Kürzlich wurde 2-Ethylhexylbenzoat als ein Produkt, welches diese Anforderungen erfüllen könnte, vorgestellt [Bohnert, Stanhope, J. Vinyl Addit. Technol. (2000), 6(3), 146-149]. Diese Verbindung hat allerdings einen vergleichsweise hohen Dampfdruck, was oft zu nicht akzeptablen Verlusten während der Verarbeitung führt.

In DE 19 62 500 wird die Verwendung eines Gemisches von längerkettigen Estern der Benzoe- und Phthalsäure zur Herstellung von Plastisolen offenbart. Zur Herstellung der Benzoesäureester wird bevorzugt 3,5,5-Trimethylhexanol eingesetzt; über die einzusetzenden Phthalsäurediester werden keine genauen Angaben gemacht.

Die Verwendung von Phthalaten mit Estergruppen, die 1 - 8 Kohlenstoffatome aufweisen, wird aus toxikologischen Gründen immer weiter eingeschränkt. Ester mit längeren Alkylseitenketten sind zwar toxikologisch günstiger eingestuft, weisen jedoch schlechtere Verarbeitungseigenschaften auf.

Die vorgenannten Weichmachersysteme weisen in PVC in ihren Geliereigenschaften, in der Kälteflexibilität und der Lagerstabilität noch Verbesserungspotential auf.

Es bestand daher die Aufgabe, neue Weichmacher für Kunststoffe wie z. B. für PVC zu finden, die eine preiswerte Rohstoffbasis haben und gleichwertige oder verbesserte Weichmachereigenschaften wie beispielsweise ein verbessertes Kälteflexibilisierungsvermögen und geringere Flüchtigkeit bei niedrigem Viskositätsniveau der entsprechenden Plastisole aufweisen.

Es wurde nun gefunden, das Benzoesäureisononylester allein oder im Gemisch mit Phthalsäureestern und/oder Adipinsäuredialkylestern und/oder Cyclohexyldicarbonsäureestern die gewünschten anwendungstechnischen Profile aufweisen.

Gegenstand der vorliegenden Erfindung sind daher Gemische isomerer Benzoesäureisononylester, wobei die durch Verseifung der isomeren Benzoesäureisononylester erhaltenen Nonylalkohole weniger als 10 Mol % 3,5,5-Trimethylhexanol enthalten.

Weitere Gegenstände der vorliegenden Erfindung sind Verfahren zur Herstellung von Gemischen isomerer Benzoesäureisononylester durch Veresterung von Benzoesäure mit Nonylalkoholen, die weniger als 10 Mol % 3,5,5-Trimethylhexanol enthalten bzw. durch Umesterung eines oder mehrerer Benzoesäurealkylester, wobei deren Alkylreste 1-8 Kohlenstoffatome enthalten mit Nonylalkoholen, die weniger als 10 Mol % 3,5,5-Trimethylhexanol enthalten.

Die Verseifung der Benzoesäureester bzw. der weiteren, im folgenden noch genannten Ester kann nach üblichen Methoden durch Umsetzung mit alkalischen Medien erfolgen (siehe z. B. Ullmann's Enzyklopädie der Technischen Chemie, 5 Ed. A 10, S. 254-260).

Die zur Herstellung der erfindungsgemäßen Gemische eingesetzten Nonylalkohole sind in der Regel Isomerengemische und werden häufig als Isononanole bezeichnet. Die erfindungsgemäßen Gemische bzw. im erfindungsgemäßen Verfahren eingesetzten Nonylalkohole weisen eine hohe Linearität auf, die durch einen Anteil von weniger als 10 Mol % (0-10),'bevorzugt weniger als 5 (0-5) Mol %, besonders bevorzugt weniger als 2 (0-2) Mol % an 3,5,5-Trimethylhexanol gekennzeichnet ist. Diese Angaben beziehen sich auf alle im folgenden genannten Gemische. Solche Gemische sind kommerziell unter den CAS-Nummern 27458-94-2, 68515-81-1, 68527-05-9 oder 68526-84-1 erhältlich.

"CAS-Nummer" bedeutet Chemical Abstracts' Registry Number. Die Isomerenverteilungen der Nonylreste können mit den üblichen, dem Fachmann geläufigen Meßmethoden wie NMR-Spektroskopie, GC- oder GC/MS-Spektroskopie ermittelt werden.

Die erfindungsgemäßen Nonylbenzoate können als Viskositätserniedriger und schnellgelierende Weichmacher verwendet werden und zeichnen sich gegenüber bekannten Systemen bei der Modifizierung von Kunststoffen wie PVC durch eine sehr vorteilhafte Kombination aus geringer Flüchtigkeit, guter Gelierfähigkeit, guter Kälteflexibilisierung und geringem Viskositätsanstieg in Plastisolen aus.
In einer Verfahrensvariante werden ein oder mehrere Benzoesäurealkylester, bevorzugt Benzoesäuremethylester, Benzoesäureethylester, Benzoesäurepropylester, Benzoesäureisobutylester, Benzoesäureamylester und/oder Benzoesäurebutylester umgeestert.

Bevorzugt werden zur Herstellung der erfindungsgemäßen Isononylbenzoate sowie der verwendeten Nonylphthalate und/oder -adipate und/oder Cyclohexyldicarbonsäureester, technische Nonanolgemische, d. h. Gemische der isomeren Alkohole, im folgenden Text als Isononanol oder Isononanolgemisch bezeichnet, eingesetzt.

Die Isomerenverteilung dieser Gemische wird durch die Art der Herstellung des verwendeten Nonylalkohols (Isononanol) bestimmt.

Isononanol wird durch Hydroformylierung von Octenen, die wiederum auf unterschiedliche Art erzeugt werden, hergestellt. Als Rohstoff hierzu dienen im allgemeinen technische C₄-Ströme, die zunächst alle isomeren C₄-Olefine neben den gesättigten Butanen und ggf. Verunreinigungen wie C₃- und C₅-Olefinen und acetylenischen Verbindungen enthalten. Durch Oligomerisierung dieses Olefingemisches erhält man vorwiegend isomere Octengemische neben höheren Oligomeren wie C₁₂- und C₁₆-Olefingemischen.

Diese Octengemische werden zu den entsprechenden Aldehyden hydroformyliert und anschließend zum Alkohol hydriert.

Die Zusammensetzung, d. h. die Isomerenverteilung der technischen Nonanolgemische ist abhängig vom Ausgangsmaterial und von den Oligomerisierungs- und Hydroformylierungsverfahren. Zur Herstellung der erfindungsgemäßen Ester können alle diese Gemische eingesetzt werden. Bevorzugte Nonanolgemische sind diejenigen, die durch Hydroformylierung von C₈-Olefingemischen, erhalten durch Oligomerisierung von im Wesentlichem linearen Butenen an Nickelträgerkatalysatoren (z. B. OCTOL-Prozeß), in Gegenwart von unmodifizierten Kobaltverbindungen und anschließender Hydrierung des entkatalysierten Hydroformylierungsgemisches gewonnen wurden. Dabei beträgt der Anteil von Isobuten im Ausgangsstoff, bezogen auf den Gesamtbutengehalt, weniger als 5 Gew-%, vorzugsweise weniger als 3 Gew-%, besonders bevorzugt weniger als 1 Gew-%. Hierdurch wird erreicht, dass der Anteil stärker verzweigter Nonanol-Isomerer, u.a. auch der des 3,5,5-Trimethylhexanols, welches sich als wenig vorteilhaft gezeigt hat, deutlich zurück gedrängt wird. Erfindungsgemäße Gemische enthalten daher unter 10, bevorzugt unter 5, besonders bevorzugt unter 3, insbesondere unter 1 Gew-% Ester des 3,5,5-Trimethylhexanols. Diese Angaben beziehen sich auf die Alkoholgemische, die sich aus der Verseifung der erfindungsgemäßen Estergemische ergeben würden.

Es sind auch Benzoesäurealkylestergemische Gegenstand der vorliegenden Erfindung, deren durch Verseifung erhältliche Alkoholmischung den Alkoholen mit den CAS-Nummern 68551-09-7, 91994-92-2, 68526-83-0, 66455-17-2, 68551-08-6, 85631-14-7 oder 97552-90-4 entsprechen.

Es handelt sich hier um Alkoholmischungen, die neben den genannten Isononylalkoholen auch Alkohole mit 7 bis 15 Kohlenstoffatome (gemäß CAS-Definition) enthalten.

Weiterhin sind Gegenstände der vorliegenden Erfindung Gemische aus den Benzoesäureisononylestern, bevorzugt die o. g. Benzoesäureisononylester mit jeweils Phthalsäuredialkylestern, bevorzugt Phthalsäurediisononylester, oder mit Adipinsäuredialkylestern, bevorzugt den Adipinsäurediisononylestern oder mit Cyclohexandicarbonsäurealkylestern, bevorzugt den Cyclohexandicarbonsäurediisononylester.

Diese erfindungsgemäßen Gemische können wie folgt definiert werden:
a) Gemische enthaltend 1-99 Gew.-% isomere Benzoesäureisononylester, wobei die durch deren Verseifung erhaltenen Nonylalkohole weniger als 10 Mol % 3,5,5-Trimethylhexanol enthalten und 1-99 Gew.-% Phthalsäuredialkylester, wobei deren Alkylreste 4 bis 13 Kohlenstoffatome enthalten. Bevorzugte Phthalsäureester sind Phthalsäurediisononylester. Besonders enthalten die durch Verseifung der Phthalsäurediisononylester erhaltenen Isononanole weniger als 10 Mol % 3,5,5-Trimethylhexanol.
b) Gemische enthaltend 1-99 Gew.-% isomere Benzoesäureisononylester, wobei die durch deren Verseifung erhaltenen Nonylalkohole weniger als 10 Mol % 3,5,5-Trimethylhexanol enthalten und 1-99 Gew.-% Adipinsäurealkylester, wobei deren Alkylreste 4 bis 13 Kohlenstoffatome enthalten.
   Bevorzugter Adipinsäurealkylester ist Adipinsäurediisononylester. Besonders bevorzugt enthalten die durch Verseifung der Adipinsäurediisononylester erhaltenen Isononanole weniger als 10 Mol % 3,5,5-Trimethylhexanol.
c) Gemische enthaltend 1-99 Gew.-% isomere Benzoesäureisononylester, wobei die durch deren Verseifung erhaltenen Nonylalkohole weniger als 10 Mol % 3,5,5-Trimethylhexanol enthalten und 1-99 Gew.-% Cyclohexandicarbonsäurealkylester, wobei deren Alkylreste 4 bis 13 Kohlenstoffatome enthalten.

Bevorzugter Cyclohexandicarbonsäuredialkylester ist Cyclohexandicarbonsäurediisononylester. Besonders bevorzugt enthalten die durch Verseifung der Cyclohexandicarbonsäureisononylester erhaltenen Isononanole weniger als 10 Mol % 3,5,5-Trimethylhexanol. Unter den Cyclohexandicarbonsäureestern sind die mit 1,2-Stellung der Carboxylgruppen wiederum bevorzugt.

Die Anteile der genannten Ester in den jeweiligen Gemischen addieren sich zu 100 %.

Erfindungsgemäße Gemische werden durch die Zusammensetzung der genannten Ester definiert, nicht durch Art oder Reihenfolge der Herstellung der Gemische. Gemische im Sinne der vorliegenden Erfindung liegen auch vor, wenn die genannten Ester im genannten Verhältnis gleichzeitig oder nacheinander mit einem weiteren Stoff wie Kunststoffen (z. B. PVC) gemischt werden.

Die Veresterung der Benzoesäure, Phthalsäure bzw. Phthalsäureanhydrid und/oder Adipinsäure und/oder Cyclohexandicarbonsäure bzw. dessen Anhydrid mit einem isomerenreinen Nonanol oder einem Isononanolgemisch zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Säure und Alkohol) und den Produkten (Ester und Wasser). Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkoholgemische niedriger als die Benzoesäure und deren Ester sieden und mit Wasser eine Mischungslücke aufweisen, werden sie häufig als Schleppmittel eingesetzt, das nach Wasserabtrennung wieder in den Prozess zurückgeführt werden kann.

Der zur Bildung des Esters eingesetzte Alkohol bzw. das isomere Alkoholgemisch, das gleichzeitig als Schleppmittel dient, wird im Überschuss, bevorzugt 5 bis 50 %, insbesondere 10 bis 30 % der zur Bildung des Esters notwendigen Menge eingesetzt.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität oft erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 180 bis 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei verminderten Druck durchgeführt. Beispielsweise wird bei der Umsetzung von Benzoesäure mit einem Gemisch isomerer Nonanole in einem Temperaturbereich von 170 °C bis 250 °C im Druckbereich von 1 bar bis 10 mbar gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.
Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und ggf. Leichtsieder, Neutralisation der vorhandenen Säuren, optional eine Wasserdampfdestillation, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann der Nonylester oder das Gemisch der Nonylester aus dem Reaktionsgemisch, gegebenenfalls nach Neutralisation des Ansatzes, destillativ abgetrennt werden.

Alternativ können die erfindungsgemäßen Nonylbenzoate durch Umesterung eines Benzoesäureesters mit Nonanol oder einem Isononanolgemisch gewonnen werden. Als Edukte werden Benzoesäureester eingesetzt, deren am O-Atom der Estergruppe gebundenen Alkykylreste 1-8 C-Atome aufweisen. Diese Reste können aliphatisch, geradkettig oder verweigt, alicyclisch oder aromatisch sein. Eine oder mehrere Methylengruppen dieser AlkylReste können durch Sauerstoff substituiert sein. Es ist zweckmäßig, dass die dem Eduktester zugrunde liegenden Alkohole niedriger sieden als das eingesetzte Nonanol oder Isononanolgemisch. Ein bevorzugter Einsatzstoff ist Benzoesäuremethylester.

Die Umesterung wird katalytisch, beispielsweise mit Brönstedt- oder Lewissäuren oder Basen, durchgeführt. Ganz gleich welcher Katalysator eingesetzt wird, es entsteht immer ein temperaturabhängiges Gleichgewicht zwischen den Einsatzstoffen (Alkylbenzoat und Nonanol oder Isononanolgemisch) und den Produkten ( Nonylester oder Nonylestergemisch und freigesetzter Alkohol). Um das Gleichgewicht zu Gunsten des Nonylesters oder des Isononylestergemisches zu verschieben, wird der aus dem Eduktester entstehende Alkohol aus dem Reaktionsgemisch abdestilliert.

Es ist auch hier zweckmäßig, Nonanol bzw. das Isononanolgemisch im Überschuss einzusetzen.
Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem Nonanol bzw. einem Isonanolgemisch hergestellt werden.

Die Katalysatorkonzentration hängt von der Art des Katalysators ab. Sie liegt üblicherweise zwischen 0,005 bis 1,0 Massen-% bezogen auf das Reaktionsgemisch.

Die Reaktionstemperaturen für die Umesterung liegen üblicherweise zwischen 100 und 220 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden.

Die erfindungsgemäßen Gemische können alleine oder in Kombination mit anderen Weichmachern in Kunststoffen eingearbeitet werden. Bevorzugte Kunststoffe sind PVC, PVB, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.
Als Vertreter der obigen Gruppen seien beispielsweise folgende Kunststoffe genannt:
Polyacrylate mit gleichen oder verschiedenen Alkylresten mit 4 bis 10 C-Atomen, gebunden am Sauerstoffatom der Estergruppe, insbesondere mit dem n-Butyl-, n-Hexyl-, n-Octyl-, Isononyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Als PVC-Typen kommen Suspensions-, Masse-, Mikrosuspensions- oder bevorzugt Emulsions-PVC in Frage. Neben den beschriebenen Estern der Cyclohexandicarbonsäure, Phthalsäure, Adipinsäure und Benzoesäure sowie weiteren Weichmachern können der Rezeptur zahlreiche weitere, dem Fachmann bekannte Komponenten beigefügt werden. Beispiele hierfür sind Füllstoffe, Pigmente, Stabilisatoren, Gleitmittel, Treibmittel, Kicker, Antioxidanzien, Biozide etc.

Bevorzugt werden die erfindungsgemäßen Gemische zur Herstellung von Plastisolen, insbesondere von denen des PVC, mit besonders vorteilhaften verarbeitungstechnischen Eigenschaften eingesetzt. Diese Plastisole können in zahlreichen Produkten wie beispielweise Kunstleder, Fußböden, Tapeten etc eingesetzt werden. Unter diesen Anwendungen besonders bevorzugt ist die Verwendung in cushion vinyl (CV)-Fußböden, hier insbesondere in der Deckschicht, wo eine weitere Verbesserung in der Fleckbeständigkeit ("Stain Resistance") bewirkt wird. Durch Verwendung der erfindungsgemäßen Gemische als Rezepturbestandteil können Plastisole mit niedriger Viskosität sowie erhöhter Lagerstabilität und gleichzeitig mit beschleunigter Gelierung und verbesserter Kälteflexibilisierung erhalten werden.

Weiterhin können die Nonylbenzoate oder die oben erwähnten erfindungsgemäßen Gemische mit Phthalaten, Adipaten und/oder Cyclohexandicarboxylaten als Flexibilisierungsmittel in Lacken, Farben, Tinten oder Klebstoffen bzw. Klebstoffkomponenten eingesetzt werden.

Die folgenden Beispiele sollen die Erfindung erläutern, ohne die Anwendungsbreite einzuschränken, die sich aus der Beschreibung und den Patentansprüchen ergibt.

### Beispiel 1:

### Herstellung von Isononylbenzoat

In einem 4-Liter-Destillationskolben mit aufgesetztem Wasserabscheider und Rückflusskühler sowie einem Probenahmestutzen und Thermometer wurden 976 g Benzoesäure (8 Mol), 1728 g Isononanol der Fa. OXENO Olefinchemie GmbH (12 Mol) und 0,59 g Butyltitanat (0,06% bezogen auf die Säuremenge) eingewogen und unter Stickstoffatmosphäre zum Sieden erhitzt. Das bei der Veresterung anfallende Reaktionswasser wurde regelmäßig abgenommen. Als die Säurezahl unter 0,1 mg KOH/g sank, (nach ca. 3 Stunden) wurde der Ansatz zunächst auf unter 60 °C abgekühlt und eine 20 cm Multifillkolonne aufgesetzt. Danach wurde der Druck auf 2 mbar verringert und zunächst der überschüssige Alkohol (etwa 120 °C) abdestilliert. Nach der Abtrennung eines Zwischenlaufes bis 140 °C, konnte in einem Bereich von 142 bis 147 °C (bei 2 mbar), gemessen am Kolonnenkopf, das Isononylbenzoat überdestilliert werden. Gaschromatographisch konnte eine Reinheit von > 99,7% ermittelt werden.
Die dynamische Viskosität des Produktes bei 20 °C betrug 8,4 mPa*s.

### Beispiel 2:

### Herstellung von 2-Ethylhexylbenzoat (Vergleichsbeispiel)

Analog zu der unter Beispiel 1 durchgeführten Prozedur wurden 12 Mol 2-Ethylhexanol mit 8 Mol Benzoesäure und Tetrabutyltitanat umgesetzt.
Nach Destillation wird 2-Ethylhexylbenzoat in einer gaschromatographisch bestimmten Reinheit von 99,7% erhalten.
Die dynamische Viskosität des Produktes bei 20 °C betrug 6,8 mPa*s.

### Beispiel 3:

### Herstellung von 3,5,5-Trimethylhexylbenzoat (Vergleichsbeispiel)

In einem 2 1 Autoklav wurden 1000 g 2,4,4-Trimethyl-1-penten (Diisobuten) von Oxeno (kann beispielsweise hergestellt werden nach DE 10106593.0) bei 135 °C unter 270 bar Synthesegasdruck 3 Stunden lang in Gegenwart eines unmodifzierten Rhodium-Katalysators hydroformyliert. Der aktive Katalysator wurde in situ aus Rhodium-Nonanoat (mit 24,8 Gew.-% Rh ) generiert. Die Rhodiumkonzentration bezogen auf Diisobuten wurde auf 20 ppm eingestellt.
Nach 3 Stunden wurde die Reaktion abgebrochen und der Autoklav auf 20 °C abgekühlt.

Der Reaktionsaustrag enthielt 93,5 Gew.-% 3,5,5-Trimethylhexanal, 2,5 Gew.-% 3,5,5-Trimethylhexanol, 3,4 Gew.-% C8-Restkohlenwasserstoffe und 0,6 Gew.-% Hochsieder.
In einer Labordestillationskolonne wurde der Reaktionsaustrag durch Destillation vom Rhodium-Katalysator befreit.
Der Rh-freie Hydroformylierungsaustrag wurde anschließend in der flüssigen Phase in einem Festbettreaktor in Gegenwart eines Cu/Cr/Ni-Katalysators bei 180 °C und 25 bar hydriert.
Nach der Hydrierung von 3,5,5-Trimethylhexanal zu dem Zielprodukt 3,5,5-Trimethylhexanol wurde der Hydrieraustrag durch gezielte Destillation von den Leichtsiedern ( C8-Kohlenwasserstoffe) befreit.
Nach der Destillation wurde ein 3,5,5-Trimethylhexanol mit einer Reinheit von über 99,5 Gew.-% erhalten.

Analog zu der unter Beispiel 1 durchgeführten Prozedur wurden 6 Mol des so hergestellten 3,5,5-Trimethylhexanol mit 4 Mol Benzoesäure und Tetrabutyltitanat umgesetzt.
Nach Destillation wird 3,5,5-Trimethylhexylbenzoat in einer gaschromatographisch bestimmten Reinheit von 99,7 % erhalten.
Die dynamische Viskosität des Produktes bei 20 °C betrug 7,9 mPa*s.

### Beispiel 4:

### Vergleich der Flüchtigkeiten von 2-Ethylhexylbenzoat, 3,5,5-Trimethylhexylbenzoat und Isononylbenzoat mittels dynamischer TGA-Messung

Um eine Aussage über die Flüchtigkeit der Produkte zu erhalten, wurden die nach den Beispielen 1 bis 3 hergestellten Benzoesäureester mit Hilfe der dynamischen TGA-Methode bezüglich ihrer Masseverluste bei höheren Temperaturen verglichen.
Zu diesem Zweck wurden etwa 40 mg einer Probe unter Stickstoffatmosphäre in einem Gerät der Marke DuPont Instruments TGA 951 in einem Temperaturbereich von 20 bis 300 °C bei einer dynamischen Temperatursteigerung von 10 K/min aufgeheizt und der jeweilige Masseverlust in % bestimmt.

In der untenstehenden Tabelle sind die nicht verdampften Anteile (= 100 % - Masseverlust in %) aufgeführt:

**Tabelle 2:**

| **Temperatur in °C** | **Isononylbenzoat** | **2-Ethylhexylbenzoat (Vergleichsbeispiel)** | **3,5,5-Trimethylhexylbenzoat (Vergleichsbeispiel)** |
|---|---|---|---|
| 140 | 98,5% | 98,1% | 93,6% |
| 170 | 93,7% | 91,1% | 72,9% |
| 200 | 75,7% | 68,2% | 9,4% |
| 230 | 24,2% | 12,4% | 0% |

Die Temperatur, bei der 50% der Probe verdampft sind, beträgt bei dem erfindungsgemäßen Isononylbenzoat 218 °C und bei der Vergleichsprobe 2-Ethylhexylbenzoat nur 213 °C. Bei 3,5,5-Trimethylhexylbenzoat sind bereits bei 184 °C 50% der Probe verdampft.
Hiermit ist die geringere Flüchtigkeit des erfindungsgemäß hergestellten Produktes in Bezug auf die Vergleichsprodukte eindeutig belegt.

### Beispiel 5:

### Herstellung von Plastisolen

In den Rezepturen 1 bis 3 sind lediglich drei schnell gelierende Weichmacher vertreten, um die Unterschiede zwischen diesen Typen stärker heraus zu arbeiten. Die Rezepturen 4-6 enthalten praxisrelevante Mischungen aus VESTINOL 9 (DINP der Fa. OXENO Olefinchemie GmbH) und Schnellgelierer in typischen Deckstrich-Formulierungen.
Die Einwaage der Komponenten ist der nachfolgenden Tabelle zu entnehmen.

**Tabelle 3: Rezepturen (Alle Angaben in phr (= Gewichtsteile pro 100 Teile PVC))**

| | **1** | **2** | **3** | **4** | **5** | **6** |
|---|---|---|---|---|---|---|
| VESTOLIT B 7021 (Emulsions-PVC) | 100 | 100 | 100 | 100 | 100 | 100 |
| VESTINOL 9 (DINP, *OXENO*) | 0 | 0 | 0 | 35 | 35 | 35 |
| Isononylbenzoat (aus Beispiel 1) | 50 | | | 15 | | |
| 2-Ethylhexylbenzoat (aus Beispiel 2) | | 50 | | | 15 | |
| 3,5,5-Trimethylhexylbenzoat (aus Beispiel 3) | | | 50 | | | 15 |
| Drapex 39 (Co-Stabilisator, *Crompton*) | 3 | 3 | 3 | 3 | 3 | 3 |
| Mark CZ 140 (Ca/Zn-Stab., *Crompton*) | 1,5 | 1,5 | 1,5 | 1,5 | 1,5 | 1,5 |

Die Weichmacher wurden vor der Zugabe auf 25 °C temperiert. Die flüssigen Bestandteile wurden zuerst, dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 6:

### Vermessung der Pastisolviskositäten

Die Messung der Viskositäten der in Beispiel 5 hergestellten Plastisole wurden in Anlehnung an die DIN 53 019 mit dem Rheometer Physica DSR 4000, welches über die Software US 200 gesteuert wird, wie folgt durchgeführt.
Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o.g. Software. Folgende Punkte wurden angesteuert:
- Eine Vorscherung von 100 s⁻¹ für den Zeitraum von 60 s, bei der keine Messwerte aufgenommen wurden
- Eine Abwärtsrampe, beginnend bei 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h, 24 h und 7 d durchgeführt. Zwischen diesen Zeitpunkten wurde die Paste bei 25°C gelagert.

In den beiden folgenden Tabellen sind für die Schergeschwindigkeiten von 1,06 s⁻¹ und 118 s⁻¹ jeweils die nach den angegebenen Lagerzeiten erhaltenen entsprechenden Viskositätswerte aufgeführt.

**Tabelle 4: Schergeschwindigkeit 1,06 s⁻¹ (Angaben der Viskositäten in Pa*s)**

| **Rezeptur** | **1** | **2** | **3** | **4** | **5** | **6** |
|---|---|---|---|---|---|---|
| **2 h** | **0,71** | 0,81 | 0,60 | **1,97** | 1,96 | 1,81 |
| **24 h** | **0,93** | 1,24 | 0,77 | **2,35** | 2,41 | 2,39 |
| **7 d** | **1,39** | 2,63 | 0,99 | **2,93** | 3,19 | 3,04 |

**Tabelle 5: Schergeschwindigkeit 118 s⁻¹ (Angaben der Viskositäten in Pa*s)**

| **Rezeptur** | **1** | **2** | **3** | **4** | **5** | **6** |
|---|---|---|---|---|---|---|
| **2 h** | **0,59** | 0,61 | 0,46 | **2,50** | 2,49 | 2,14 |
| **24 h** | **0,73** | 0,86 | 0,57 | **2,91** | 2,93 | 2,93 |
| **7 d** | **1,00** | 1,51 | 0,72 | **3,45** | 3,60 | 3,51 |

Mit den in den Tabellen 4 und 5 aufgeführten Messwerten soll gezeigt werden, dass sich Plastisole mit dem erfindungsgemäßen Isononylbenzoat von ihrem Viskositätsniveau her nur unwesentlich von den beiden Benzoaten aus dem Stand der Technik unterscheiden. Insbesondere in Abmischungen mit DINP unterscheiden sich die drei Benzoate kaum.

### Beispiel 7:

### Messung der Geliereigenschaften

Die Untersuchung des Gelierverhaltens der Plastisole wurde in einem Oszillationsviskosimeter der Marke Bohlin CVO (Meßsystem PP20), welches schubspannungsgesteuert betrieben wurde, vorgenommen.
Folgende Parameter wurden eingestellt:
- Modus:: Temperatur-Gradient
Start-Temperatur: 25°C
End-Temperatur: 180°C
Heiz/Kühlrate: 2°C/min
Temperatur nach der Messung: 25°C
Oszillations-Frequenz: 2 Hz
Verzögerungszeit: 1 s
Wartezeit: 15 s
Kontinuierliche Oszillation: an
Automatische Schubspannungsvorgabe: an
Startschubspannung: 0,3 Pa
Soll-Deformation: 0,002
Spaltweite 0,5 mm

### Durchführung des Meßvorganges:

Auf die untere Meßsystemplatte wurde mit dem Spatel ein Tropfen des zu messenden Plastisols (Rezepturen 1-3 aus Beispiel 5) luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet.
Aufgetragen wurde die "komplexe Viskosität" des Plastisols in Abhängigkeit von der Temperatur. Ein Einsetzen des Geliervorganges ist in einem plötzlichen starken Anstieg der komplexen Viskosität zu erkennen. Je früher dieser Viskositätsanstieg einsetzt, desto besser ist die Gelierfähigkeit des Systems.

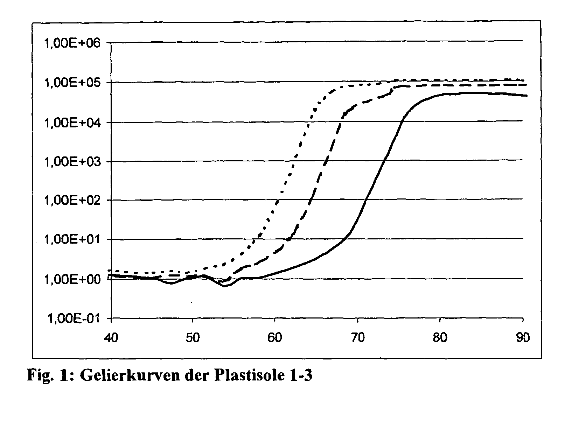

In Fig. 1 ist der für das Einsetzen der Gelierung relevante Ausschnitt des Viskositäts-Temperatur-Verlaufs ("Gelierkurve") aufgeführt (siehe Fig. 1). Die Y-Achse zeigt die komplexen Viskositäten in Pa·s, die X-Achse die Temperaturen in °C. Die durchgezogene Linie bezeichnet Plastisol 3 (3,5,5-Trimethylhexalbenzoat), die gepunktete Linie bezeichnet Plastisol 2 (2-Ethylhexalbenzoat) und die gestrichelte Linie bezeichnet Plastisol 1 (Isononylbenzoat).

In dieser Darstellung, in der aus Gründen der Übersichtlichkeit nur die Formulierungen der Schnellgelierer ohne DINP (1 bis 3) enthalten sind, ist zu erkennen, dass das Isononylbenzoat-Plastisol deutlich früher als das entsprechende Plastisol mit 3,5,5-Trimethylhexylbenzoat mit dem starken Viskositätsanstieg, d.h. mit der Gelierung, einsetzt. Die etwas geringere Geliertemperatur des Plastisols auf Basis 2-Ethylhexylbenzoat ist in Übereinstimmung mit den Erwartungen, dass mit abnehmender Kettenlänge die Geliertemperatur sinkt. Überraschend ist hierbei, dass der Effekt der unterschiedlichen Verzweigung bei gleichem Molekulargewicht hier deutlich signifikanter zu Tage tritt als der Effekt beim Übergang von 2-Ethylhexyl- (C8) zu Isononylbenzoat (C9).

### Beispiel 7: Beurteilung der Kälteeigenschaften durch Torsionsschwingungsanalyse

Die unter Beispiel 5 hergestellten Plastisole wurden auf Release-Papier in einem üblichen Laborgelierofen (Mathis LTSV) bei 200 °C zwei Minuten lang zu 1 mm dicken Folien ausgezogen und geliert.
Danach wurden von den Folien 60 mm lange, 80 mm breite und 1 mm dicke Stücke ausgestanzt und von diesen in einem Torsionspendel vom Typ MYRENNE ATM III nach DIN EN ISO 6721 (Teil 2) bei Temperaturen von -100 °C bis +100 °C und einer Frequenz von 1 s⁻¹ jeweils die Steifigkeit G' und der Verlustmodul G" bestimmt.
Aus dem Maximum von G" ließ sich die Glasübergangstemperatur T_{G} bestimmen. Diese ist ein Maß für die Flexibilität bei tiefen Temperaturen.
Die Glasübergangstemperaturen der aus den Plastisolen 1 - 6 aus Beispiel 5 hergestellten Folien können Tabelle 6 entnommen werden:

**Tabelle 6:**

| Plastisol-Nr. | **1** | **2** | **3** | **4** | **5** | **6** |
|---|---|---|---|---|---|---|
| T_{G} in °C | **-49** | -47 | -39 | **-35** | -35 | -33 |

Während die mit Isononylbenzoat und 2-Ethylhexylbenzoat erreichbaren Glasübergangstemperaturen insbesondere in Mischungen auf einem ähnlichen Niveau liegen, ist jedoch Isononylbenzoat dem 3,5,5-Trimethylhexylbenzoat deutlich zu bevorzugen.

Zusammenfassend lässt sich sagen, dass Rezepturen auf der Basis von Isononylbenzoat verglichen mit denen auf Basis von 3,5,5-Trimethylhexylbenzoat bei praktisch gleichem Viskositätsniveau deutliche Vorteile in Bezug auf Gelierfähigkeit, Kälteflexibilisierung und Flüchtigkeit zeigen.

Gegenüber auf 2-Ethylhexylbenzoat basierenden Rezepturen kann insbesondere die Flüchtigkeit reduziert und die Kälteflexibilisierung nochmals verbessert werden.

## Patentansprüche

1. Gemische isomerer Benzoesäureisononylester,
**dadurch gekennzeichnet**,
dass die durch Verseifung der isomeren Benzoesäureisononylester erhaltenen Nonylalkohole weniger als 10 Mol % 3,5,5-Trimethylhexanol enthalten.

2. Gemische enthaltend 1-99 Gew.-% isomere Benzoesäureisononylester gemäß Anspruch 1, wobei die durch deren Verseifung erhaltenen Nonylalkohole weniger als 10 Mol % 3,5,5-Trimethylhexanol enthalten und 1-99 Gew.-% Phthalsäuredialkylester, wobei deren Alkylreste 4 bis 13 Kohlenstoffatome enthalten.

3. Gemisch nach Anspruch 2,
**dadurch gekennzeichnet**,
dass als Phthalsäurealkylester Phthalsäurediisononylester eingesetzt werden.

4. Gemisch nach Anspruch 3,
**dadurch gekennzeichnet**,
dass die durch Verseifung der Phthalsäurediisononylester erhaltenen Nonylalkohole weniger als 10 Mol % 3,5,5-Trimethylhexanol enthalten.

5. Gemische enthaltend 1-99 Gew.-% isomere Benzoesäureisononylester gemäß Anspruch 1, wobei die durch deren Verseifung erhaltenen Nonylalkohole weniger als 10 Mol % 3,5,5-Trimethylhexanol enthalten und 1-99 Gew.% Adipinsäurealkylester, wobei deren Alkylreste 4 bis 13 Kohlenstoffatome enthalten.

6. Gemisch nach Anspruch 5,
**dadurch gekennzeichnet**,
dass als Adipinsäurealkylester Adipinsäurediisononylester eingesetzt werden.

7. Gemisch nach Anspruch 6,
**dadurch gekennzeichnet**,
dass die durch Verseifung der Adipinsäurediisononylester erhaltenen Nonylalkohole weniger als 10 Mol % 3,5,5-Trimethylhexanol enthalten.

8. Gemische enthaltend 1-99 Gew.-% isomere Benzoesäureisononylester gemäß Anspruch 1, wobei die durch deren Verseifung erhaltenen Nonylalkohole weniger als 10 Mol % 3,5,5-Trimethylhexanol enthalten und 1-99 Gew.-% Cyclohexandicarbonsäurealkylester, wobei deren Alkylreste 4 bis 13 Kohlenstoffatome enthalten.

9. Gemisch nach Anspruch 8,
**dadurch gekennzeichnet**,
dass als Cyclohexandicarbonsäurealkylester Cyclohexandicarbonsäurediisononylester eingesetzt werden.

10. Gemisch nach Anspruch 9,
**dadurch gekennzeichnet**,
dass die durch Verseifung der Cyclohexandicarbonsäurediisononylester erhaltenen Nonylalkohole weniger als 10 Mol % 3,5,5-Trimethylhexanol enthalten.

11. Verfahren zur Herstellung isomerer Benzoesäureisononylester, hergestellt durch Veresterung von Benzoesäure mit Nonylalkoholen, die weniger als 10 Mol % 3,5,5-Trimethylhexanol enthalten.

12. Verfahren zur Herstellung isomerer Benzoesäurenonylester, hergestellt durch Umesterung eines oder mehrerer Benzoesäurealkylester, wobei deren Alkylreste 1 bis 8 Kohlenstoffatome aufweisen, mit Nonylalkoholen die weniger als 10 Mol % 3,5,5-Trimethylhexanol enthalten.

13. Verwendung der Gemische nach einem der Ansprüche 1 bis 10 oder 11 und 12 als Weichmacher in Kunststoffen.

14. Verwendung der Gemische nach einem der Ansprüche 1 bis 10 oder 11 und 12 als Weichmacher in PVC.

15. Verwendung der Gemische nach einem der Ansprüche 1 bis 10 oder 11 und 12 als Weichmacher in PVC-Plastisolen.

16. Verwendung der Gemische nach einem der Ansprüche 1 bis 10 oder 11 und 12 in Farben und Lacken.

17. Verwendung der Gemische nach einem der Ansprüche 1 bis 10 oder 11 und 12 in Klebstoffen oder Klebstoffkomponenten.

## Claims

1. Mixtures of isomeric isononyl benzoates,
**characterized in that**
the nonyl alcohols obtained by saponifying the isomeric isononyl benzoates comprise less than 10 mol% of 3,5,5-trimethylhexanol.

2. Mixtures comprising from 1 to 99% by weight of isomeric isononyl benzoates according to Claim 1, where the nonyl alcohols obtained by saponifying these benzoates comprise less than 10 mol% of 3,5,5-trimethylhexanol, and comprising from 1 to 99% by weight of dialkyl phthalates whose alkyl radicals contain from 4 to 13 carbon atoms.

3. Mixture according to Claim 2,
**characterized in that**
the alkyl phthalates used comprise diisononyl phthalates.

4. Mixture according to Claim 3,
**characterized in that**
the nonyl alcohols obtained by saponifying the diisononyl phthalates comprise less than 10 mol% of 3,5,5-trimethylhexanol.

5. Mixtures comprising from 1 to 99% by weight of isomeric isononyl benzoates according to Claim 1, where the nonyl alcohols obtained by saponifying these benzoates comprise less than 10 mol% of 3,5,5-trimethylhexanol, and comprising from 1 to 99% by weight of alkyl adipates whose alkyl radicals contain from 4 to 13 carbon atoms.

6. Mixture according to Claim 5,
**characterized in that**
the alkyl adipates used comprise diisononyl adipates.

7. Mixture according to Claim 6,
**characterized in that**
the nonyl alcohols obtained by saponifying the diisononyl adipates comprise less than 10 mol% of 3,5,5-trimethylhexanol.

8. Mixtures comprising from 1 to 99% by weight of isomeric isononyl benzoates according to Claim 1, where the nonyl alcohols obtained by saponifying these benzoates comprise less than 10 mol% of 3,5,5-trimethylhexanol, and comprising from 1 to 99% by weight of alkyl cyclohexanedicarboxylate whose alkyl radicals contain from 4 to 13 carbon atoms.

9. Mixture according to Claim 8,
**characterized in that**
the alkyl cyclohexanedicarboxylates used comprise diisononyl cyclohexanedicarboxylates.

10. Mixture according to Claim 9,
**characterized in that**
the nonyl alcohols obtained by saponifying the diisononyl cyclohexanedicarboxylates comprise less than 10 mol% of 3,5,5-trimethylhexanol.

11. Process for preparing isomeric isononyl benzoates prepared by esterifying benzoic acid with nonyl alcohols which comprise less than 10 mol% of 3,5,5-trimethylhexanol.

12. Process for preparing isomeric nonyl benzoates prepared by transesterifying one or more alkyl benzoates whose alkyl radicals have from 1 to 8 carbon atoms with nonyl alcohols which comprise less than 10 mol% of 3,5,5-trimethylhexanol.

13. Use of the mixtures according to any of Claims 1 to 10, 11, or 12 as plasticizers in plastics.

14. Use of the mixtures according to any of Claims 1 to 10, 11, or 12 as plasticizers in PVC.

15. Use of the mixtures according to any of Claims 1 to 10, 11, or 12 as plasticizers in PVC plastisols.

16. Use of the mixtures according to any of laims 1 to 10, 11, or 12 in paints and coatings.

17. Use of the mixtures according to any of Claims 1 to 10, 11, or 12 in adhesives or components of adhesives.

## Revendications

1. Mélanges d'esters isomères isononyliques de l'acide benzoïque, **caractérisés en ce que** les alcools nonyliques obtenus par saponification des esters isomères isononyliques de l'acide benzoïque contiennent moins de 10% en mole de 3,5,5-triméthylhexanol.

2. Mélanges contenant 1-99% en poids d'esters isomères isononyliques de l'acide benzoïque selon la revendication 1, les alcools nonyliques obtenus par leur saponification contenant moins de 10% en mole de 3,5,5-triméthylhexanol et 1-99% en poids d'esters dialkyliques de l'acide phtalique, leurs radicaux alkyle contenant 4 à 13 atomes de carbone.

3. Mélange selon la revendication 2, **caractérisé en ce qu'**on utilise, comme esters alkyliques de l'acide phtalique, des esters diisononyliques de l'acide phtalique.

4. Mélange selon la revendication 3, **caractérisé en ce que** les alcools nonyliques obtenus par saponification des esters diisononyliques de l'acide phtalique contiennent moins de 10% en mole de 3,5,5-triméthylhexanol.

5. Mélanges contenant 1-99% en poids d'esters isomères isononyliques de l'acide benzoïque selon la revendication 1, les alcools nonyliques obtenus par leur saponification contenant moins de 10% en mole de 3,5,5-triméthylhexanol et 1-99% en poids d'esters alkyliques de l'acide adipique, leurs radicaux alkyle contenant 4 à 13 atomes de carbone.

6. Mélange selon la revendication 5, **caractérisé en ce qu'**on utilise, comme esters alkyliques de l'acide adipique, des esters diisononyliques de l'acide adipique.

7. Mélange selon la revendication 6, **caractérisé en ce que** les alcools nonyliques obtenus par saponification des esters diisononyliques de l'acide adipique contiennent moins de 10% en mole de 3,5,5-triméthylhexanol.

8. Mélanges contenant 1-99% en poids d'esters isomères isononyliques de l'acide benzoïque selon la revendication 1, les alcools nonyliques obtenus par leur saponification contenant moins de 10% en mole de 3,5,5-triméthylhexanol et 1-99% en poids d'esters alkyliques de l'acide cyclohexanedicarboxylique, leurs radicaux alkyle contenant 4 à 13 atomes de carbone.

9. Mélange selon la revendication 8, **caractérisé en ce qu'**on utilise, comme esters alkyliques de l'acide cyclohexanedicarboxylique, des esters diisononyliques de l'acide cyclohexanedicarboxylique.

10. Mélange selon la revendication 9, **caractérisé en ce que** les alcools nonyliques obtenus par saponification des esters diisononyliques de l'acide cyclohexanedicarboxylique contiennent moins de 10% en mole de 3,5,5-triméthylhexanol.

11. Procédé pour la préparation d'esters isomères isononyliques de l'acide benzoïque, préparés par estérification d'acide benzoïque avec des alcools nonyliques qui contiennent moins de 10% en mole de 3,5,5-triméthylhexanol.

12. Procédé pour la préparation d'esters isomères nonyliques de l'acide benzoïque, préparés par transestérification d'un ou de plusieurs esters alkyliques de l'acide benzoïque, leurs radicaux alkyle présentant 1 à 8 atomes de carbone, avec des alcools nonyliques qui contiennent moins de 10% en mole de 3,5,5-triméthylhexanol.

13. Utilisation des mélanges selon l'une quelconque des revendications 1 à 10 ou 11 et 12 comme plastifiant dans des matériaux synthétiques.

14. Utilisation des mélanges selon l'une quelconque des revendications 1 à 10 ou 11 et 12 comme plastifiant dans le PVC.

15. Utilisation des mélanges selon l'une quelconque des revendications 1 à 10 ou 11 et 12 comme plastifiant dans les plastisols de PVC.

16. Utilisation des mélanges selon l'une quelconque des revendications 1 à 10 ou 11 et 12 dans les encres et les laques.

17. Utilisation des mélanges selon l'une quelconque des revendications 1 à 10 ou 11 et 12 dans les adhésifs ou les composants pour adhésifs.
